# EUROPEAN PATENT SPECIFICATION

(11) **EP 3 838 302 B1**
(45) Date of publication and mention of the grant of the patent: **23.08.2023**
(21) Application number: 20215133.8
(22) Date of filing: 17.12.2020
(51) Int. Cl.: A61L 15/32, A61L 15/42, A61L 15/44

(54) **SANDWICH AND COMPOSITE COLLAGEN SPONGE FOR CONTROLLED RELEASE OF ACTIVE SUBSTANCES, AND METHOD OF PREPARATION THEREOF**
SANDWICH- UND VERBUNDKOLLAGENSCHWAMM ZUR KONTROLLIERTEN FREISETZUNG VON WIRKSTOFFEN UND VERFAHREN ZU DESSEN HERSTELLUNG
SANDWICH ET ÉPONGE COMPOSITE À BASE DE COLLAGÈNE POUR LA LIBÉRATION CONTRÔLÉE DE SUBSTANCES ACTIVES ET LEUR PROCÉDÉS DE FABRICATION

(30) Priority: 17.12.2019 CZ 20190777
(43) Date of publication of application: 23.06.2021
(60) Divisional application: 22170293.9
(73) Proprietor: Vseobecná fakultní nemocnice v Praze, 12800 Praha 2, Nové Mesto (CZ); Ústav struktury a mechaniky hornin AV CR, v.v.i., 182 00 Praha 8, Liben (CZ); Ceské Vysoké Ucení Technické V Praze Fakulta Strojni, 160 00 Praha 6, Dejvice (CZ)
(72) Inventor: Grus, Tomas, 25210 Mnisek pod Brdy (CZ); Suchy, Tomas, 19000 Praha 9, Strizkov (CZ); Supova, Monika, 14900 Praha 4, Haje (CZ); Chlup, Hynek, 79807 Dobrochov (CZ); Hartinger, Jan, 14000 Praha 4 (CZ)
(74) Representative: Lunzarová, Lucie

(56) References cited:
- WO-A1-2010/084481
- US-A1- 2003 133 967
- US-A1- 2016 038 611
- WACHOL-DREWEK Z ET AL: "Comparative investigation of drug delivery of collagen implants saturated in antibiotic solutions and a sponge containing gentamicin", BIOMATERIALS, ELSEVIER, AMSTERDAM, NL, vol. 17, no. 17, 1 September 1996 (1996-09-01), pages 1733-1738, XP004032983, ISSN: 0142-9612, DOI: 10.1016/0142-9612(96)87654-X
- HARTINGER JAN MIROSLAV ET AL: "Vancomycin-releasing cross-linked collagen sponges as wound dressings", BOSNIAN JOURNAL OF BASIC MEDICAL SCIENCES, vol. 21, no. 1, 29 November 2019 (2019-11-29), pages 61-70, XP055775372, SARAJEVO, BOSNIA ISSN: 1512-8601, DOI: 10.17305/bjbms.2019.4496
- POWELL ET AL: "Influence of electrospun collagen on wound contraction of engineered skin substitutes", BIOMATERIALS, ELSEVIER, AMSTERDAM, NL, vol. 29, no. 7, 3 December 2007 (2007-12-03), pages 834-843, XP022394092, ISSN: 0142-9612, DOI: 10.1016/J.BIOMATERIALS.2007.10.036
- RHO K S ET AL: "Electrospinning of collagen nanofibers: Effects on the behavior of normal human keratinocytes and early-stage wound healing", BIOMATERIALS, ELSEVIER, AMSTERDAM, NL, vol. 27, no. 8, 1 March 2006 (2006-03-01), pages 1452-1461, XP027950899, ISSN: 0142-9612 [retrieved on 2006-03-01]

## Description

### Field of the invention

The present invention relates to a method of preparation of a degradable sandwich collagen sponge with hemostatic effects, controlled degradation time and capable of controlled local release of active substances, for example antibiotics, for use in wound dressings in surgery, orthopedics, traumatology and plastic surgery, comprising a low porous collagen core and highly porous collagen sponge as peripheral parts.

### Background of the invention

Bleeding on larger areas during surgery is a relatively common and very serious problem and in extreme cases, it can be life-threatening. Another serious problem is the penetration of an infection into the body. During postoperative administration of antibiotics, there is a problem of a systemic load, often an insufficient penetration thereof into the surgical wound and in some cases, a systemic toxicity can occur.

One solution is to use a locally antiseptic non-adhesive material. In addition to its essential local antiseptic function, this material can also have both local hemostatic and anaesthetic properties.

In biomaterial engineering, collagens isolated from large mammals such as cow, pig or horse are widely used. Their disadvantage are the potential allergic reactions that afflict 3-4% of the population. The solution is offered in the use of fish collagen, which shows excellent biocompatibility. In addition, the isolation of collagen from fish skin does not pose such a risk of transmitting various diseases as in the case of extraction from pig or bovine skin (BSE, PrPSc). Fish collagen shows lower reproducibility in terms of its structural properties compared to collagens isolated from large mammals. This drawback can be overcome by its chemical crosslinking with carboimide-based compounds commonly used in collagen chemistry. The crosslinking conditions affect the resulting degree of crosslinking of the collagen material, which in turn has a great influence on its absorbency and degradation properties in the physiological environment. Crosslinked collagen resists ionizing radiation well, so it can be sterilized with commonly used doses of 25 kGy. Using the lyophilization process, it is possible to further create a three-dimensional material with a given porosity.

US2003/133967 A1 discloses a multilayer biocompatible sheet material comprising a first and second layer comprising reconstituted matrices of biocompatible collagen, which layers are physically adhered along at least a portion of a surface of each of said layers and wherein at least one said layer is capable of absorbing sufficient fluids to form an expanded matrix capable of promoting cell growth.

Collagen material can also be prepared in the form of nanofiber layers by electrospinning. Their huge advantage is the large active surface, which can effectively bind large amounts of chemical agents and at the same time, it is also a positive factor for cell adhesion, proliferation and growth.

The materials thus prepared have generally proved as controlled-elution drug carriers. The drug can be incorporated into the collagen directly into the solution before the given processing procedures or by additional impregnation of the ready material with a solvent, which does not degrade the collagen structure and at the same time is able to dissolve the desired drug. Additional impregnation has proven to be the most effective method, as there is no premature drug release during the collagen material processing.

The ability to process collagen into various final forms (layers, sponges, etc.), its easy and economical availability (waste material in the food industry) together with its biocompatible and hemocoagulating properties make it an optimal candidate for use in hemostatic sponges for covering surgical and postoperative wounds.

The most commonly used antibiotic in collagen dosage forms is gentamicin. In clinical practice, these products are used especially in dentistry, abdominal surgery and traumatology. Recent clinical studies in the field of cardiac surgery also confirm the effectiveness of this approach in the prevention of postoperative infections. There are also studies concerning the use of collagen matrices with tetracycline and metronidazole in dental surgery. Collagen matrices releasing chlorhexidine in the oral cavity are used to treat periodontitis. In the vast majority of studies and clinically used products, the basic matrix is mammalian collagen (bovine or equine). In addition to mammalian collagen, sea fish collagen is in rare cases used for this purpose - only for the application of chlorhexidine in dentistry. In vitro data and animal experiment data for ciprofloxacin are also available. It's clear from the above that fish collagen, namely collagen from sea fish, not freshwater fish, is used clinically as a drug release matrix only marginally for the chlorhexidine treatment of periodontitis. It is not used for antibiotics treatment. Another interesting option that is not currently available would be the use of vancomycin in collagen sponge. In clinical practice, vancomycin is used mainly in the local treatment of fractures - vancomycin-impregnated beads and bone allografts have been used in combination with aminoglycosides containing cement to replace the hip joint. Vancomycin-impregnated collagen sponges have not been studied. Due to the fact that vancomycin, as well as aminoglycosides, insufficiently penetrates into tissues, systemic application for the treatment of postoperative wound infections is problematic. Local application with sustained release would be particularly suitable for this antibiotic because, unlike aminoglycosides, its efficacy depends not only on the concentration achieved, but also on the time when the concentration exceeds the MIC. The value best correlated with the clinical response in systemic therapy of *Staphylococcus aureus* infections is AUC / MIC. If collagen dosage forms ensured long-term adequate local concentrations of vancomycin in the wound, it would be possible in this way to abandon the systemic use of other backup antibiotics (ceftaroline, linezolid) and their saving for more serious infections.

Local application is especially suitable for antibiotics with low tissue distribution, which correspond to aminoglycosides, vancoymcin and nitrofurantoin. We are newly placing these substances in the collagen matrix of freshwater fish collagen, which is readily available material with lower immunogenicity than collagen obtained from mammals. We achieve different levels of retardation depending on the nature of the antibiotic, e.g., rapid release and high concentrations of gentamicin, or slow release of vancomycin - these characteristics correspond to different mechanisms of antibiotics action and different target pharmacokinetic parameters (concentration x time-dependent antibiotics).

Other disadvantages of existing collagen-based materials include difficult handling because these materials in contact with the body environment lose cohesion and tend to tear and disintegrate during handling.

### Summary of the invention

The degradable collagen sponge for controlled release of active substances according to the invention overcome the disadvantages of collagen-based hemostatic dressing materials to a significant extent. The invention is directed to a method of preparation of the sandwich collagen sponge comprising peripheral layers formed by a highly porous sponge which contains type I collagen and a low porous core containing type I collagen, characterized in that 4 to 8% w/w dispersion of type I collagen in water is first allowed to swell at 5-15 °C for 1-3 hours, then the mixture is homogenized, after homogenization the mixture is dispensed into moulds, frozen at -60 to -90 °C for 3 - 8 hours and lyophilized to form a low porous core, which is subsequently impregnated with 0.1 - 3% w/w dispersion of type I collagen for 1-3 hours at 15-25 °C, then the mixture is frozen again at -60 to -90 °C for 3 - 8 hours and lyophilized to form a sandwich collagen sponge, after which the sponge is stabilized by chemical crosslinking which is carried out by dipping the sponge in a solution of 95% w/w ethanol and water with N-(3-dimethylaminopropyl)-N-ethylcarbodiimide hydrochloride and N-hydroxysuccinimide at 25 - 40 °C for 2 4 hours, washed, frozen at -15 to -30 °C for 5 7 hours and lyophilized.

The peripheral layers comprise a highly porous collagen sponge which contains type I collagen and has an open porosity in the range of 70 - 90% and a mean pore size of 50 - 200 µm, wherein at least 50% of the pore volume consists of pores with size more than 80 µm. Preferably, the collagen is derived from the skin of freshwater fish, where the immune responses are significantly lower than that of collagen of mammalian origin, in particular from the tissues of large mammals. The highly porous sponge can be further impregnated with at least one active substance, which is arranged in the pores of the sponge and the ratio of collagen to the total amount of active substances in the collagen sponge is 40 - 70% w/w of collagen to 30 - 60% w/w of active substances. The active substance is preferably selected from the group including an antibiotic, an anesthetic, for example bupivacaine, an antiaggregant, an antiviral, for example ganciclovir or acyclovir, or a mixture thereof, preferably an antibiotic, for example vancomycin, gentamicin, rifampicin, nitrofurantoin, or a mixture thereof. The sponge preferably has a thickness in the range of 0.5 to 5 cm, for example 1 cm.

The sponges according to the invention are characterized by controlled degradation, controlled porosity and the ability to release active substances, in particular antibiotics.

The highly porous collagen sponge has a high open porosity rate of 70-90% by ' volume, a pore size in the range of 50 - 200 µm, and is designed to exhibit a sustainable local release of active substances, the total amount of which is 30 - 60% w/w. The controlled release of active substances, such as antibiotics, and the controlled degradation of collagen sponge is achieved by forming optimal amounts of cross-links in the collagen molecule during its preparation, either physically, e.g. by irradiation, or chemically, preferably by application of N-(3-dimethylaminopropyl)-N- ethylcarbodiimide hydrochloride (EDC) and N-hydroxysuccinimide (NHS). The degradation of the non-crosslinked sponge without or with active substances, for example with antibiotics, can be expected in the order of 2 to 7 days. The sponge crosslinked for 2 hours degrades within 2 weeks. The sponge crosslinked for 3 hours degrades within 4 weeks. The sponge crosslinked for 4 hours degrades within 6 weeks. These data relate to the sponge, which is based on the method of preparation using 1% w/w collagen dispersion, EDC and NHS in a weight ratio of 0.625 g EDC and 0.156 g NHS per 1 g of collagen. When using a different initial concentration, the degradation times may vary, but it can be determined by one skilled in the art by routine experimentation.

The controlled degradation time, which can be selected based on the desired sponge application time, subsequently affects the controlled release time of active substances, such as antibiotics (see Examples 2-4). The sponge (using a 1% w/w collagen dispersion in the preparation) crosslinked for 2 hours releases antibiotics within ten days. The sponge crosslinked for 3 hours releases antibiotics within fifteen days. The sponge crosslinked for 4 hours releases antibiotics within twenty days.

In the preventive use of sponge with antibiotics, a sponge with a degradation time of up to two weeks and with a release of antibiotics of up to ten days, preferably four days, is preferably used. In cases of risk of inflammation, in potentially infectious terrain (e.g., groin surgery, near the genitals, or laparoscopic removal of the gallbladder or appendix), a sponge with a degradation time of up to four weeks and a release of antibiotics of up to fifteen days, preferably ten days, is preferably used. In cases of infection caused by the presence of prosthetic material, where there is a high risk of infection, a sponge with a degradation time of up to eight weeks, preferably six weeks and an antibiotic release time of up to fifty days, preferably twenty days according to susceptibility to cultured infectious agents is preferably used.

The desired porosity is achieved on the one hand by the concentration of the collagen dispersion from which the sponge is prepared, and on the other hand by the freezing point of the resulting sponge before lyophilization and subsequent impregnation with the active substance. The more concentrated the collagen dispersion, the smaller the pores, and the lower the freezing temperature, the smaller the pores.

The basic benefit of the present invention is the fact that the used collagen isolated from the skin of freshwater fish has hemostatic effects and its application is not associated with the risk of an allergic reaction or other adverse immune response of the organism. Another significant benefit of the present invention is the fact, that microstructured surface with a large specific surface area and sufficient open porosity allows to deposit the biologically active substance by impregnating the collagen sponge, e.g., in an antibiotic solution, according to the expected extent of injury or damage before surgery, but also at the discretion of the surgeon during the surgery. At the same time, the resorbable material enables its gradual safe absorption into the surrounding tissue, and thus the gradual effective release of biologically active substances deposited in it.

Disclosed herein is a sandwich collagen sponge for controlled release of active substances, which contains type I collagen and its structure consists of a low porous core and highly porous peripheral layers, the low porous core having an open porosity in the range of 70 - 90% and a mean pore size from 20 to 80 µm, wherein at least 50% of the pore volume consists of pores with size below 50 µm, and the highly porous peripheral layer having an open porosity in the range of 70 - 90%, preferably 80% to 90%, and a mean pore size from 50 to 200 µm, wherein at least 50% of the pore volume consists of pores with size more than 80 µm. In a preferred embodiment, the volume of the core is from 10 to 90%, preferably from 30 to 70%, most preferably from 40 to 60 %. The sandwich sponge may further contain at least one active substance, wherein the ratio of collagen to the total amount of active substances in the collagen sponge is 40 - 70% w/w of collagen to 30 - 60% w/w of active substances. The active substance is preferably selected from the group including an antibiotic, an anaesthetic, an antiaggregant, an antiviral, or a mixture thereof, preferably an antibiotic or a mixture of antibiotics, for example selected from the group including vancomycin, gentamicin, rifampicin, nitrofurantoin. The whole sandwich preferably has a thickness in the range of 0.5 to 5 cm, for example 1 cm.

Sponges prepared from 1% w/w collagen dispersion can absorb up to 30 times their weight of fluid during the first week. Full soaking occurs after only four hours. The ability to soak decreases over time due to an ongoing degradation. A solid core prepared from 5% w/w collagen dispersion can absorb up to 15 times its weight of fluid during the first week.

In all embodiments of the invention, the type I collagen is preferably collagen isolated from the skin of freshwater fish, for example from the skin of carp.

In the preparation of a highly porous collagen sponge, the weighed amount of collagen is mixed with deionized water in a concentration of 0.1 - 3% w/w, preferably 0.5 to 1% w/w of collagen, wherein the collagen is allowed to swell at a temperature of 5 to 15 °C for 1 to 3 hours. After homogenization, the resulting dispersion is frozen at -60 to -90 °C for 3 to 8 hours and lyophilized until a constant weight is reached. After lyophilization, the stability of the collagen sponge is preferably increased by chemical crosslinking with EDC and NHS, then the sponge is re-frozen at -15 to 30 °C for 5 to 7 hours and lyophilized. To ensure the controlled release of the active substance into the body, the last step of the preparation is the impregnation of the sponge by alcohol-based solution containing a weighed amount of active substances, e.g., antibiotics, preferably selected from the group including vancomycin, gentamicin, rifampicin, nitrofurantoin, or a combination thereof, so that their final amount in the solution is 30 - 60% w/w. The impregnated sponge can then be used as it is, or it can be frozen and lyophilized. Alternatively, the impregnation with the selected active substances can be carried out just before the application of the sponge, for which an aqueous solution of the active substances is used or the active substances are mixed with saline and the sponge is applied immediately or after evaporation of the liquid phase, i.e., after constant weight of the sponge is reached. By this procedure, the sponge is obtained with a final amount of collagen of 40 - 70% w/w and 30 - 60% w/w of active substances.

In the preparation of a sandwich collagen sponge, the weighed amount of collagen is mixed with deionized water in a concentration of 4 - 8% w/w collagen, the collagen is allowed to swell at 5 to 15 °C for 1 to 3 hours. After homogenization, the resulting dispersion is frozen at -60 to -90 °C for 3 - 8 hours and lyophilized until a constant weight is reached to form a low porous solid core. This rigid, low porous core is further impregnated with 0.1-3% w/w collagen dispersion and thus left for 1-3 hours at 15 - 25 °C, frozen at -60 to -90 °C for 3 to 8 hours and lyophilized until a constant weight is reached. In this way, a sandwich collagen sponge is prepared, wherein the inner part consists of a low-porous core and the peripheral parts consist of highly porous layers. The final volume amount of the low porous core in the sandwich sponge is 10 - 90%, preferably 30 - 70%, most preferably 40 - 60%. After lyophilization, the stability of the sandwich collagen sponge is increased physically or chemically, by chemical crosslinking with EDC and NHS, after which the sponge is re-frozen at -15 to -30 °C for 5 to 7 hours and lyophilized. Furthermore, the sponge can be impregnated with an alcohol-based solution, e.g., 95% w/w an aqueous ethanol solution containing a weighed amount of active ingredients, wherein the active ingredients may be an antibiotic, an anesthetic, an antiaggregant, an antiviral, or a mixture thereof, preferably an antibiotic selected from the group including vancomycin, gentamicin, rifampicin, nitrofurantoin, or combinations thereof, that their final amount is 30 - 60% w/w. The impregnated sponge can then be frozen and lyophilized. The impregnation with the selected antibiotics can be carried out only before its application, for which an aqueous solution of antibiotics is used or the antibiotics are mixed with physiological solution and the sponge is applied after their evaporation, i.e., after constant weight of the sponge is reached. By this procedure, a sponge is obtained with a final amount of collagen of 40 - 70% w/w and 30 - 60% w/w of antibiotics.

The sandwich sponge is designed to exhibit a sustainable local release of active substances, the total amount of which is 30 - 60% w/w. The controlled release of active substances and the controlled degradation of the collagen sponge is achieved by forming optimal amounts of cross-links in the collagen molecule, either physically, e.g., by irradiation, or chemically, preferably by applying N-(3-dimethylaminopropyl)-N-ethylcarbodiimide hydrochloride (EDC) and N-hydroxysuccinimide (NHS) in its preparation.

The degradation time of the sandwich sponge is mainly controlled by the time and the temperature of the stabilization (crosslinking). The controlled degradation time, which can be selected on the basis of the desired sponge application time, also affects the controlled release time of active substances, for example antibiotics, whereby the release from the peripheral layers is rapid, while the release from the core is longer.

Thanks to the method of sandwich collagen sponge preparation, the individual layers do not separate, the layers remain connected even when handled in the body environment and do not separate from each other. At the same time, the sandwich sponge according to the invention is characterized by an elastic memory and flexural stiffness, easy handling (no tearing) and the possibility of reposition.

The shorter the crosslinking time, the faster the degradation of the composite and the shorter the release time of the active substances.

The active substance for the sandwich is preferably selected from the group including an antibiotic, anesthetic, e.g., bupivacaine, antiaggregant, antiviral, e.g., ganciclovir or acyclovir, or a mixture thereof, preferably an antibiotic, e.g., vancomycin, gentamicin, rifampicin, nitrofurantoin, or a mixture thereof.

The required degree of crosslinking of type I collagen can be quantified on the basis of collagen amino acid analysis, i.e., by quantifying amino acid residues with free carboxyl and amine groups, which allow new cross-links forming during the application of EDC / NHS. Control of collagen breakdown, and thus controlled release of active substances from collagen sponges, can be ensured by applying the optimal concentration of EDC quantified on the basis of the mass concentration of these amino acids, such as aspartic acid and glutamine (carboxyl groups) and lysine and hydroxylisin (amine groups), which are present in collagen in the order of tens to hundreds of mmol, depending on various factors. Based on the molar concentration of aspartic acid, glutamine (carboxyl groups), lysine and hydroxylisine (amine groups) in collagen, the weight amount of EDC is determined as 8-11 times the sum of the molar amount of these amino acids. Neither EDC, nor NHS is part of the newly formed bond, but is converted to a water-soluble urea derivative.

The sandwich sponge of the invention have the following advantages:
- programmable (controlled) sponge decomposition
- programmable (controlled) release of active substances
- elastic memory, flexural stiffness
- easier sponge handling, possibility of reposition (does not tear, does not disintegrate)
- reduced risk of an allergic reaction or other adverse immune response.

The collagen sponge according to the invention is preferably used especially preventively, in order to rapidly release the active substances.

### Brief description of the drawings

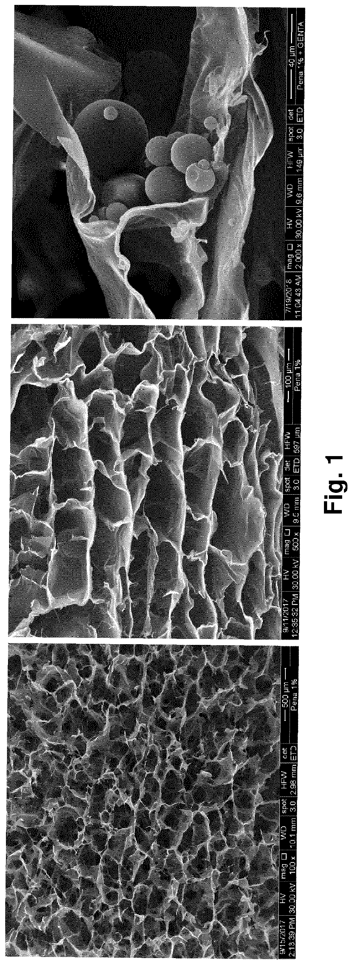

Figure 1 shows a collagen sponge prepared from 1% w/w collagen dispersion without antibiotics (left: 100-fold magnification, middle: 500-fold magnification) and after impregnation with gentamicin (far right, 2000-fold magnification).

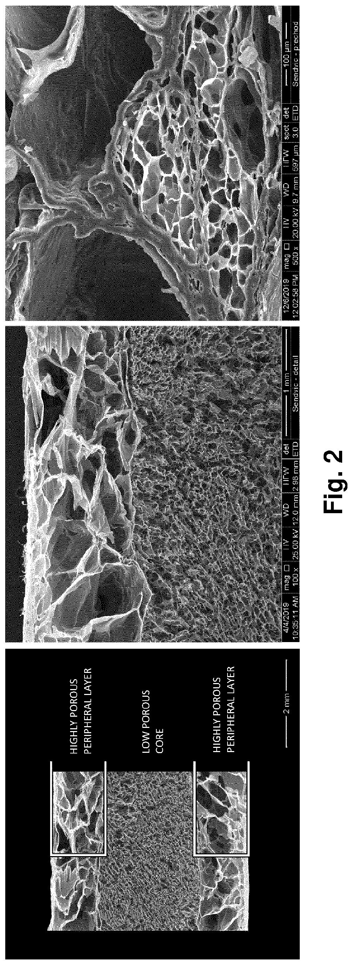

Figure 2 shows a sandwich collagen sponge with a low porous core prepared from 5% w/w collagen dispersion, and with highly porous peripheral parts prepared from 1% w/w collagen dispersion, preparation according to Example 11. The image on the far right shows the transition interface between the core and the periphery.

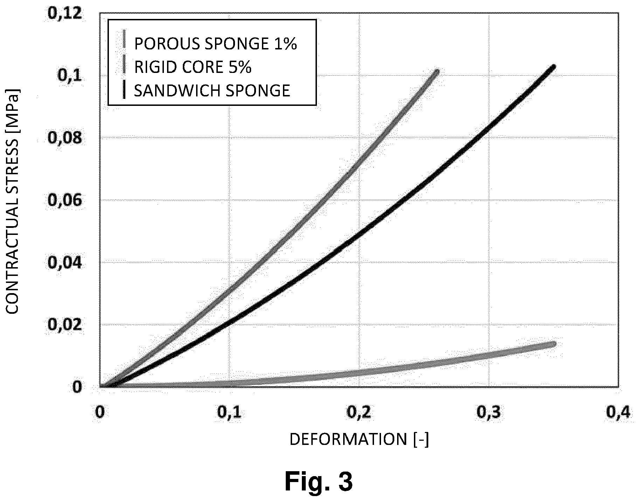

Figure 3 shows a graph of the dependence of the stress on the deformation for the porous sponge alone, the rigid core alone and for the sandwich sponge (preparation according to Example 11). The graph shows a several fold higher resistance to deformation of the sandwich sponge compared to the sponge itself.

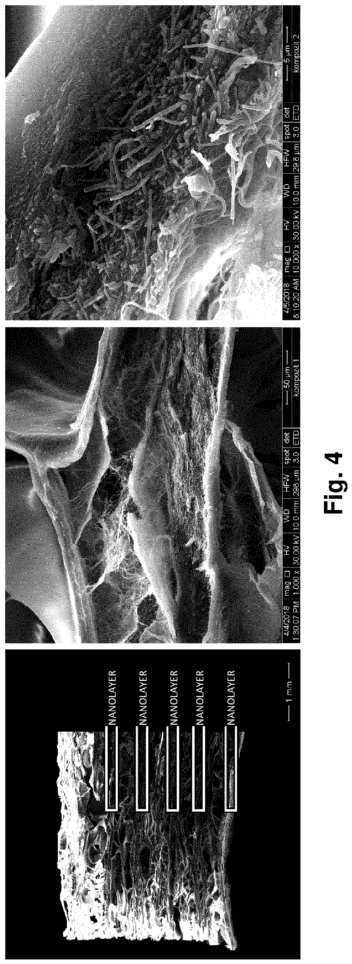

Figure 4 shows a nanostructured highly porous composite sponge prepared by saturation of five nanofiber layers with 1% w/w collagen dispersion. The image in the middle illustrates the transition between the fibers and the porous structure of the sponge. The image on the far right shows the detail of the saturation of the nanofiber layer with dispersion.

### Examples of embodiments of the invention

### Definitions

Collagen sponge in the context of the present invention is synonymous with the term "collagen foam", i.e., a porous collagen-based material.

The term "open porosity" or "open pores" refers to a space that communicates with the surface of the product, i.e., with the external environment. These can be individual pores connected to the surface, but also interconnected pores, at least one of which also communicates with the surface of the product.

The term "closed porosity" or "closed pores" refers to the so-called solitary pores or pores that are not connected to the outer surface of the product.

The open porosity was determined by micro-CT (microcomputer tomography) using a SkyScan 1272 micro-CT device (Bruker micro-CT, Kontich, Belgium).

The term "highly porous" in the context of the present invention refers to those sponges which have an open porosity in the range of 70% to 90%, preferably 80% to 90%, and a pore size in the range of 50 to 200 µm, wherein at least 50% of the pore volume, or 50% by volume of the pores, consists of pores with the size more than 80 µm, preferably more than 100 µm.

The term "low porosity" in the context of the present invention refers to those sponges which have an open porosity in the range of 70% to 90% and a pore size in the range of 20 to 80 µm, wherein at least 50% of the pore volume, or 50% by volume of the pores, consist of pores with size below 50 µm.

The term "at least 50%" refers to 50 to 100%.

The pore size was determined using scanning electron microscopy (SEM) with a Quanta 450 Microscope (FEI, Hillsboro, Oregon, USA) under high vacuum.

### Example 1

In the preparation of a highly porous collagen sponge in general, the weighed amount of collagen, preferably isolated from the skin of freshwater fish, for example carp, is mixed with deionized water in a concentration of 0.1 - 3% w/w, preferably 0.5 up to 1% w/w collagen. After swelling of the collagen at 5 - 15 °C for 1-3 hours, the mixture is homogenized twice for 1 - 20 minutes at 5 - 15,000 rpm with a 20-minute delay at 15-25 °C. The resulting dispersion is then dispensed into moulds, frozen at -60 to -90 °C for 3 - 8 hours and lyophilized until a constant weight is reached. After lyophilization, the stability of the collagen sponge is increased by dipping in a solution of 95% w/w ethanol and water with EDC and NHS at 25 - 40 °C, preferably at 37 °C, for 2 - 4 hours. 0.5-1.5 g of EDC and 0.125 - 0.750 g NHS mixed with 140 - 160 ml 95% w/w ethanol solution with water were used per 1 g of collagen. After collagen crosslinking, the sponge is washed with 0.5 - 1.5 M disodium hydrogen phosphate for at least 30 minutes and further washed for at least 20 minutes in distilled water, frozen at -15 to -30 °C for 5 - 7 hours and lyophilized. The last step of the preparation is the impregnation of the sponge with 95% w/w a solution of ethanol with water containing a weighed amount of an antibiotic selected from the group including vancomycin, gentamicin, rifampicin, nitrofurantoin, or a combination thereof so that their final amount is 60% w/w. The impregnated sponge can then be frozen at -60 to -90 °C, lyophilized and cut to desired size. The sponge may also be impregnated with selected antibiotics right before application, using an aqueous solution of antibiotics or mixing the antibiotics with saline. Subsequently, the sponge is applied either soaked in the impregnating solution, or dry, i.e., only after the liquid phase has evaporated from the sponge. In this way, a sponge is obtained with a final amount of collagen of 40% w/w and 60% w/w of antibiotics.

### Example 2

A weighed amount of collagen is mixed with deionized water in a concentration of 1% w/w collagen. After swelling of the collagen at 10 °C for 2 hours, the mixture is homogenized twice for 10 minutes at 10,000 rpm with a 20-minute delay at 20 °C. The resulting dispersion is then dispensed into moulds, frozen at -70 °C for 6 hours and lyophilized until a constant weight is reached. After lyophilization, the stability of the collagen sponge is increased by dipping in a solution of 95% w/w ethanol and water with EDC and NHS at 30 °C for 2 hours. 1 g of EDC and 0.250 g of NHS mixed with 150 ml of 95% w/w ethanol solution with water were used per 1 g of collagen. After collagen crosslinking, the sponge is washed with 1 M disodium hydrogen phosphate for at least 30 minutes and further washed for at least 20 minutes in distilled water, frozen at -20 °C for 6 hours and lyophilized. The last step of the preparation is the impregnation of the sponge with 95% w/w a solution of ethanol with water containing a weighed amount of gentamicin so that its final amount in solution is 45% w/w. The impregnated sponge is then frozen at -70 °C, lyophilized and cut to desired size. In this way, a sponge is obtained with the final amount of collagen of 55% w/w and 45% w/w of antibiotics.

### Example 3

The procedure is the same as in Example 2, but the crosslinking time is 3 hours.

### Example 4

The procedure is the same as in Example 2, but the crosslinking time is 4 hours.

### Example 5

The procedure is as in Example 2, except for the last preparation step, i.e., the impregnation of the sponge with a solution containing the active substance. The sponge is frozen at -70 °C, lyophilized and cut to desired size. In this way, a sponge consisting of pure collagen is obtained.

### Example 6

A weighed amount of collagen is mixed with deionized water at a concentration of 1.5% w/w collagen. After swelling of the collagen at 15 °C for 1 hour, the mixture is homogenized twice for 15 minutes at 8,000 rpm with a 20-minute delay at 15 °C. The resulting dispersion is then dispensed into moulds, frozen at -80 °C for 4 hours and lyophilized until a constant weight is reached. After lyophilization, the stability of the collagen sponge is increased by dipping in a solution of 95% w/w ethanol and water with EDC and NHS at 25 °C for 3 hours. 0.7 g of EDC and 0.2 g of NHS mixed with 140 ml of 95% w/w ethanol solution with water were used per 1 g of collagen. After collagen crosslinking, the sponge is washed with 0.8 M disodium hydrogen phosphate for at least 30 minutes and further washed for at least 20 minutes in distilled water, frozen at -30 °C for 5 hours and lyophilized. The last step of the preparation is the impregnation of the sponge with 95% w/w a solution of ethanol with water containing a weighed amount of vancomycin so that its final amount in solution is 45% w/w. The impregnated sponge is then frozen at -80 °C, lyophilized and cut to desired size. In this way, a sponge is obtained with a final amount of collagen of 55% w/w and 45% w/w of antibiotics.

### Example 7

A weighed amount of collagen is mixed with deionized water at a concentration of 3% w/w collagen. After swelling of the collagen at 12 °C for 2.5 hours, the mixture is homogenized twice for 20 minutes at 15,000 rpm with a 20-minute delay at 25 °C. The resulting dispersion is then dispensed into moulds, frozen at -90 °C for 3 hours and lyophilized until a constant weight is reached. After lyophilization, the stability of the collagen sponge is increased by dipping in a solution of 95% w/w ethanol and water with EDC and NHS at 40 °C for 2 hours. 1.4 g of EDC and 0.7 g of NHS mixed with 160 ml of 95% w/w ethanol solution with water were used per 1 g of collagen. After collagen crosslinking, the sponge is washed with 1.5 M disodium hydrogen phosphate for at least 30 minutes and further washed for at least 20 minutes in distilled water, frozen at -30 °C for 5 hours and lyophilized. The last step of the preparation is the impregnation of the sponge with 95% w/w a solution of ethanol with water containing a weighed amount of a 1: 1 mixture of vancomycin and gentamicin so that their final amount was 30% w/w. The impregnated sponge is then frozen at -90 °C, lyophilized and cut to desired size. In this way, a sponge is obtained with a final amount of collagen of 70% w/w and 30% w/w of antibiotics.

### Example 8

A weighed amount of collagen is mixed with deionized water at a concentration of 0.5% w/w collagen. After swelling of the collagen at 15 °C for 3 hours, the mixture is homogenized twice for 20 minutes at 5,000 rpm with a 20-minute delay at 25 °C. The resulting dispersion is then dispensed into moulds, frozen at -60 °C for 8 hours and lyophilized until a constant weight is reached. After lyophilization, the stability of the collagen sponge is increased by dipping in a solution of 95% w/w ethanol and water with EDC and NHS at 25 °C for 4 hours. 1.2 g of EDC and 0.7 g of NHS mixed with 160 ml of 95% w/w ethanol solution with water were used per 1 g of collagen. After collagen crosslinking, the sponge is washed with 0.5 M disodium hydrogen phosphate for at least 30 minutes and further washed for at least 20 minutes in distilled water, frozen at -15 °C for 7 hours and lyophilized. The last step of the preparation is the impregnation of the sponge with 95% w/w a solution of ethanol with water containing a weighed amount of a mixture of vancomycin, gentamicin and nitrofurantoin in a ratio of 1: 1: 1 so that their final amount is 30% w/w. The impregnated sponge is then frozen at -60 °C, lyophilized and cut to desired size. In this way, a sponge is obtained with a final amount of collagen of 70% w/w and 30% w/w of antibiotics.

### Example 9

### Maximum degradation times of collagen sponge and maximum release times of active substances (here antibiotics)

Tab. 1 The achieved maximum degradation time of collagen sponge using EDC in a concentration of 0.5-1.5 g (per 1 g of collagen) according to the used temperature and exposure time in the crosslinking system 95% w/w ethanol solution with water EDC and NHS. The ratio of NHS to EDC is 1: 4 by weight. The basic parameters of the procedure are according to example 2.

| Achieved maximum degradation time | crosslinking time at 15-25 °C | crosslinking time at 30-40 °C | maximum antibiotic release time |
|---|---|---|---|
| 2 weeks | 6 hours | 2 hours | 10 days |
| 4 weeks | 9 hours | 3 hours | 15 days |
| 6 weeks | 12 hours | 4 hours | 20 days |

### Example 10

In the preparation of a sandwich collagen sponge in general, the weighed amount of collagen, preferably isolated from the skin of freshwater fish, such as carp, is mixed with deionized water, after swelling of the collagen at 5-15 °C for 1-3 hours, the mixture is homogenized twice for 1 - 20 minutes at 5 - 15,000 rpm with a 20-minute delay at 15 - 25 °C. The concentration of the collagen dispersion for the preparation of the core is 4 - 8% w/w. The concentration for the preparation of the peripheral parts is 0.1 - 3% w/w, preferably 0.1 - 1% w/w. In the first step, the resulting 4 - 8% w/w dispersion is dispensed into moulds, frozen at -60 to -90 °C for 3 - 8 hours and lyophilized until a constant weight is reached. In the second step, the solid core is impregnated with 0.1 - 3% w/w dispersion in the mould and thus left for 1-3 hours at 15-25 °C, frozen at -60 to -90 °C for 3 - 8 hours and lyophilized until a constant weight, to obtain a sandwich collagen sponge with a low porous core and highly porous peripheral layers. After lyophilization, the stability of the sandwich collagen sponge is increased by dipping in a solution of 95% w/w ethanol and water with EDC and NHS at 25 to 40 °C, preferably 30 to 40 °C, most preferably at 37 °C for 2 - 4 hours. 0.5 - 1.5 g of EDC and 0.125 - 0.750 g NHS mixed with 140 - 160 ml 95% w/w ethanol solution with water were used per 1 g of collagen. After collagen crosslinking, the sponge is washed with 0.5-1.5 M disodium hydrogen phosphate for at least 30 minutes and further washed for at least 20 minutes in distilled water, frozen at -15 to -30 °C for 5 - 7 hours and lyophilized. The final volume amount of the low porous core in the sandwich sponge is 40 to 60%. The last step of the preparation according to the preferred embodiment is the impregnation of the sandwich sponge with 95% w/w a solution of ethanol with water containing a weighed amount of active substances, for example antibiotics selected from the group including vancomycin, gentamicin, rifampicin, nitrofurantoin, or a combination thereof, so that their final amount is 30 to 60% w/w. The impregnated sponge can then be frozen and lyophilized. The sandwich sponge can also be impregnated with selected antibiotics only before its application, for which an aqueous solution of antibiotics is used or the antibiotics are mixed with physiological solution and the sponge is applied after their evaporation, i.e., after a constant weight of the sponge is reached. By this procedure, a sponge is obtained with a final amount of collagen of 40 - 60% w/w and 40 - 60% w/w of antibiotics.

### Example 11

In the preparation of a sandwich collagen sponge, the weighed amount of collagen is mixed with deionized water in a concentration of 5% w/w collagen, after swelling of the collagen at 10 °C for 2 hours, the mixture is homogenized twice for 10 minutes at 10,000 rpm with a 20-minute delay at 20 °C. The resulting 5% w/w dispersion is dispensed into moulds, frozen at -80 °C for 6 hours and lyophilized until a constant weight is reached to form a solid, low porous core. In the second step, the solid core is impregnated with 1% w/w dispersion in the mould, the volume ratio of the two dispersions (dispersion for the preparation of the core and the dispersion for the preparation of the peripheral parts) being 1:1, and thus left for 2 hours at 20 °C, frozen at -80 °C for 6 hours and lyophilized until a constant weight is reached to obtain a sandwich sponge consisting of a low porous core and highly porous peripheral layers. After lyophilization, the stability of the sandwich collagen sponge is increased by dipping in a solution of 95% w/w ethanol and water with EDC and NHS at 37 °C for 2 hours. 1 g of EDC and 0.250 g of NHS mixed with 150 ml of 95% w/w ethanol solution with water were used per 1 g of collagen. After collagen crosslinking, the sponge is washed with 1 M disodium hydrogen phosphate for at least 30 minutes and further washed for at least 20 minutes in distilled water, frozen at -20 °C for 6 hours and lyophilized. The final volume amount of the low porous core in the sandwich sponge is 50%. The last step of the preparation is the impregnation of the sandwich sponge with 95% w/w a solution of ethanol with water containing a weighed amount of antibiotics selected from the group including vancomycin, gentamicin, rifampicin, nitrofurantoin, or a combination thereof so that their final amount is 45% w/w. The impregnated sponge can then be frozen and lyophilized. In this way, a sponge is obtained with a final amount of collagen of 55% w/w and 45% w/w of antibiotics.

### Example 12

The procedure is the same as in Example 11, but the crosslinking time is 3 hours.

### Example 13

The procedure is the same as in Example 11, but the crosslinking time is 4 hours.

### Example 14

The procedure is as in Example 11, except for the last preparation step, i.e., the sponge is not impregnated with a solution containing the active substance. The sponge is frozen at -70 °C, lyophilized and cut to desired size. In this way, a sandwich sponge consisting of pure collagen is obtained.

### Example 15

A weighed amount of collagen is mixed with deionized water at a concentration of 4% w/w, after swelling of the collagen at 15 °C for 1 hour, the mixture is homogenized twice for 15 minutes at 8,000 rpm with a 20-minute delay at 15 °C. In the first step, the resulting 4% w/w dispersion is dispensed into moulds, frozen at -75 °C for 4 hours and lyophilized until a constant weight is reached to give a solid core. In the second step, the solid core is impregnated with 0.6% w/w dispersion in the mould, the ratio of the core dispersion to the peripheral layer dispersion being 2: 3, and thus left for 1 hour at 25 °C, frozen at -75 °C for 4 hours and lyophilized until a constant weight is reached to obtain a sandwich sponge consisting of a low porous core and highly porous peripheral layers. After lyophilization, the stability of the sandwich collagen sponge is increased by dipping in a solution of 95% w/w ethanol and water with EDC and NHS at 37 °C for 3 hours. 0.7 g of EDC and 0.2 g of NHS mixed with 140 ml of 95% w/w ethanol solution with water were used per 1 g of collagen. After collagen crosslinking, the sponge is washed with 0.8 M disodium hydrogen phosphate for at least 30 minutes and further washed for at least 20 minutes in distilled water, frozen at -30 °C for 5 hours and lyophilized. The final volume amount of the low porous core in the sandwich sponge is 40%. The last step of the preparation is the impregnation of the sandwich sponge with 95% w/w a solution of ethanol with water containing a weighed amount of antibiotics selected from the group including vancomycin, gentamicin, rifampicin, nitrofurantoin, or a combination thereof so that their final amount is 30% w/w. The impregnated sponge can then be frozen and lyophilized. In this way, a sponge is obtained with a final amount of collagen of 70% w/w and 30% w/w of antibiotics.

### Example 16

A weighed amount of collagen is mixed with deionized water at a concentration of 7% w/w, after swelling of the collagen at 12 °C for 2.5 hours, the mixture is homogenized twice for 20 minutes at 15,000 rpm with a 20-minute delay at 25 °C. In the first step, the resulting 7% w/w dispersion is dispensed into moulds, frozen at -85 °C for 3 hours and lyophilized until a constant weight is reached to give a solid core. In the second step, the solid core is impregnated with 1% w/w dispersion in the mould (the ratio of the dispersion for the preparation of the core to the dispersion for the preparation of the peripheral layers is 3: 2) and thus left for 2.5 hours at 25 °C, frozen at -85 °C for 3 hours and lyophilized until a constant weight is reached to obtain a sandwich sponge consisting of a low porous core and highly porous peripheral layers. After lyophilization, the stability of the sandwich collagen sponge is increased by dipping in a solution of 95% w/w ethanol and water with EDC and NHS at 37 °C for 3 hours. 1.4 g of EDC and 0.7 g of NHS mixed with 160 ml of 95% w/w ethanol solution with water were used per 1 g of collagen. After collagen crosslinking, the sponge is washed with 1.5 M disodium hydrogen phosphate for at least 30 minutes and further washed for at least 20 minutes in distilled water, frozen at -30 °C for 5 hours and lyophilized. The final volume amount of the low porous core in the sandwich sponge is 60%. The last step of the preparation is the impregnation of the sandwich sponge with 95% w/w a solution of ethanol with water containing a weighed amount of antibiotics selected from the group including vancomycin, gentamicin, rifampicin, nitrofurantoin, or a combination thereof so that their final amount is 30% w/w. The impregnated sponge can then be frozen and lyophilized. In this way, a sponge is obtained with a final amount of collagen of 70% w/w and 30% w/w of antibiotics.

### Example 17

A weighed amount of collagen is mixed with deionized water at a concentration of 8% w/w, after swelling of the collagen at 20 °C for 3 hours, the mixture is homogenized twice for 20 minutes at 6,000 rpm with a 20-minute delay at 25 °C. In the first step, the resulting 8% w/w dispersion is dispensed into moulds, frozen at -80 °C for 3 hours and lyophilized until a constant weight is reached, to obtain a solid core. In the second step, the solid core is impregnated with 1.5% w/w dispersion in the mould (the ratio of the core preparation dispersion to the peripheral layers dispersion is 7: 3) and thus left for 2 hours at 25 °C, frozen at -80 °C for 3 hours and lyophilized until a constant weight is reached to obtain sandwich sponge consisting of a low porous core and highly porous peripheral layers. After lyophilization, the stability of the sandwich collagen sponge is increased by dipping in a solution of 95% w/w ethanol and water with EDC and NHS at 37 °C for 2 hours. 1.4 g of EDC and 0.35 g of NHS mixed with 160 ml of 95% w/w ethanol solution with water were used per 1 g of collagen. After collagen crosslinking, the sponge is washed with 1.5 M disodium hydrogen phosphate for at least 30 minutes and further washed for at least 20 minutes in distilled water, frozen at -30 °C for 5 hours and lyophilized. The final volume amount of the low porous core in the sandwich sponge is 70%. The last step of the preparation is the impregnation of the sandwich sponge with 95% w/w a solution of ethanol with water containing a weighed amount of antibiotics selected from the group including vancomycin, gentamicin, rifampicin, nitrofurantoin, or a combination thereof so that their final amount is 30% w/w. The impregnated sponge can then be frozen and lyophilized. In this way, a sponge is obtained with a final amount of collagen of 70% w/w and 30% w/w of antibiotics.

### Example 18

Table 2 summarizes the maximum achieved degradation times of the sandwich collagen sponge and the maximum antibiotic release times, crosslinking times and temperatures, the preparation of the sandwich sponge being performed according to Example 11 with variable final volume amounts of the low porous core in the sandwich sponge 40 %, 50%, 60%, i.e., with variable ratios of initial dispersions (2: 3, 1: 1, 3: 2).

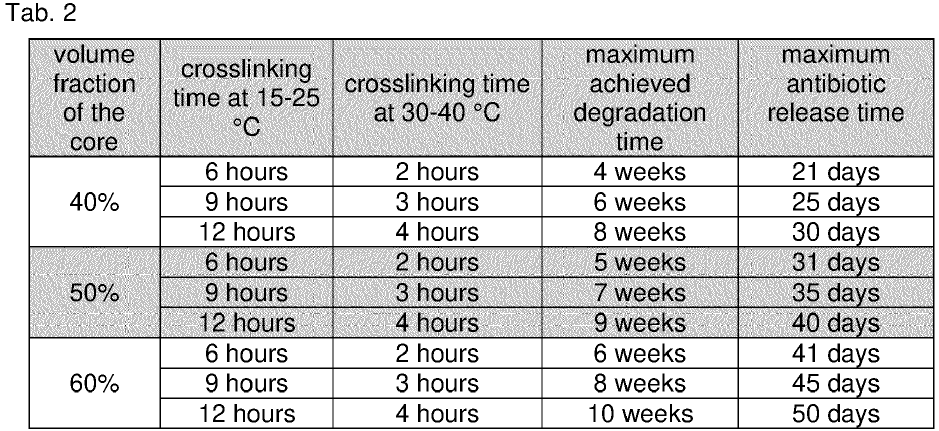

### Example 19 (not according to the invention)

In the preparation of a nanostructured highly porous composite collagen sponge in general, a nanofiber collagen layer is first prepared by electrostatic spinning of 5 - 11% w/w, preferably 6 - 10% w/w collagen solution containing polyethylene oxide (PEO; Mr 400,000 - 900,000) in a ratio of PEO to collagen in the range of 0.05 to 0.11, phosphate buffer and ethanol (1: 1 to 1: 2 by volume). The spinning takes place in an electrostatic field of 200 - 300 kV.m⁻¹ with a dosage of 100 - 200 µl / min and a temperature of not more than 37 °C. The stability of the layer is increased by dipping in a solution of 95% w/w ethanol and water with EDC and NHS at 25 - 40 °C, preferably 37 °C for 2 - 4 hours. 0.5-1.5 g of EDC and 0.125 - 0.750 g NHS mixed with 140 - 160 ml 95% w/w ethanol solution with water were used per 1 g of collagen. After collagen crosslinking, the layer is washed with 0.5 - 1.5 M disodium hydrogen phosphate for at least 30 minutes and further washed for at least 20 minutes in distilled water. In this process, the auxiliary polymer is washed out. Next, the layer is frozen at -15 to -30 °C for 5 - 7 hours and lyophilized. In the next step, the spun layer, or several superimposed layers, left in 0.1 - 3% w/w collagen dispersion for 1-3 hours at 15-25 °C. Under these conditions, the fiber layer is / layers are saturated with the collagen dispersion, followed by freezing at -60 to -90 °C for 3 - 8 hours and lyophilizing until a constant weight is reached to obtain a collagen composite sponge. After lyophilization, the stability of the collagen composite sponge is increased by dipping in a solution of 95% w/w ethanol and water with EDC and NHS at 25 to 40 °C for 2 - 4 hours. 0.5-1.5 g of EDC and 0.125 - 0.750 g NHS mixed with 140 - 160 ml 95% w/w ethanol solution with water were used per 1 g of collagen. After collagen crosslinking, the sponge is washed with 0.5-1.5 M disodium hydrogen phosphate for at least 30 minutes and further washed for at least 20 minutes in distilled water, frozen at -15 to -30 °C for 5 - 7 hours and lyophilized. The final volume amount of the collagen nanofiber layer in the composite is 20 to 60%. The last, optional preparation step is the impregnation of the composite sponge with 95% w/w a solution of ethanol with water containing a weighed amount of active substances, for example antibiotics, preferably selected from the group including vancomycin, gentamicin, rifampicin, nitrofurantoin, or a combination thereof, so that their final amount is 30 to 60% w/w. The impregnated sponge can then be frozen and lyophilized. The impregnation with the selected antibiotics can be carried out right before its application, for which an aqueous solution of antibiotics is used or the antibiotics are mixed with saline and the sponge is applied after evaporation, i.e., after reaching a constant sponge weight. In this way, a sponge is obtained with a final amount of collagen of 40 to 70% w/w and 30 to 60% w/w of antibiotics.

### Example 20 (not according to the invention)

In the preparation of a nanostructured highly porous composite collagen sponge, the nanofibrous collagen layer is prepared first by electrostatic spinning of 8% w/w a solution of collagen with phosphate buffer and ethanol (1: 1 by volume), to which polyethylene oxide is added in a weight ratio of 0.08 to collagen (Mr 700,000). The spinning takes place in an electrostatic field of 270 kV.m⁻¹, dosing of 150 µl.min⁻¹ and at the temperature of 25 °C. The stability of the layer is again increased by dipping in a solution of 95% w/w ethanol and water with EDC and NHS at 37 °C for 2 hours. 1 g of EDC and 0.250 g of NHS mixed with 150 ml of 95% w/w ethanol solution with water were used per 1 g of collagen. After crosslinking the collagen, the layer is washed with 1 M disodium hydrogen phosphate for at least 30 minutes and further washed for at least 20 minutes in distilled water. In this process, the auxiliary polymer is washed out. Next, the layer is frozen at -25 °C for 6 hours and lyophilized. In the next step, the spun layer is left in 1% w/w collagen dispersion for 2 hours at 25 °C. Under these conditions, the spun layer is saturated with collagen dispersion, followed by freezing at -80 °C for 5 hours and lyophilization until a constant weight is reached. After lyophilization, the stability of the collagen composite sponge is increased by dipping in a solution of 95% w/w ethanol and water with EDC and NHS at 37 °C for 2 hours. 1 g of EDC and 0.250 g of NHS mixed with 150 ml of 95% w/w ethanol solution with water were used per 1 g of collagen. After collagen crosslinking, the sponge is washed with 1 M disodium hydrogen phosphate for at least 30 minutes and further washed for at least 20 minutes in distilled water, frozen at -25 °C for 6 hours and lyophilized. The volume ratio of fiber layer and dispersion is 2: 3, and therefore the volume ratio of fiber layer and sponge in the resulting composite is 2: 3, i.e., the final volume amount of collagen nanofiber layer in the composite is 40%. The last step of the preparation is the impregnation of the composite sponge with 95% w/w a solution of ethanol with water containing a weighed amount of vancomycin so that its final amount is 45% w/w. The impregnated composite is then frozen at -25 °C for 6 hours and lyophilized. In this way, a composite sponge is obtained with a final amount of collagen of 55% w/w and 45% w/w of antibiotics.

### Example 21 (not according to the invention)

In the preparation of a nanostructured highly porous composite collagen sponge, the nanofiber collagen layer is prepared first as in Example 20. Stabilization, freezing and lyophilization are also conducted as in Example 2. The resulting layer is cut into smaller pieces, for example 5 x 10 cm, which are placed on top of each other (5 nanofiber layers lie on top of each other) and inserted into 1% w/w collagen dispersion for 2 hours at 25 °C. Under these conditions, the spun layers are saturated with collagen dispersion, followed by freezing at -70 °C for 6 hours and lyophilization until a constant weight is reached. After lyophilization, the stability of the collagen composite sponge is increased by dipping in a solution of 95% w/w ethanol and water with EDC and NHS at 37 °C for 2 hours. 1 g of EDC and 0.250 g of NHS mixed with 150 ml of 95% w/w ethanol solution with water were used per 1 g of collagen. After collagen crosslinking, the sponge is washed with 1 M disodium hydrogen phosphate for at least 30 minutes and further washed for at least 20 minutes in distilled water, frozen at -25 °C for 6 hours and lyophilized. The volume ratio of all fiber layers to dispersion is 2:3, and therefore the volume ratio of fiber layers and sponge in the resulting composite is 2: 3, i.e., the final volume amount of collagen nanofiber layers in the composite is 40%. The last step of the preparation is the impregnation of the composite sponge with 95% w/w a solution of ethanol with water containing a weighed amount of a mixture of vancomycin and gentamicin so that their final amount is 30% w/w. The impregnated composite is then frozen at -25 °C for 6 hours and lyophilized. In this way, a composite sponge is obtained with a final amount of collagen of 70% w/w and 30% w/w of antibiotics.

### Example 22 (not according to the invention)

The procedure is the same as in Example 20, but the crosslinking (stabilization) time is 3 hours.

### Example 23 (not according to the invention)

The procedure is the same as in Example 20, but the crosslinking (stabilization) time is 4 hours.

### Example 24 (not according to the invention)

In the preparation of a nanostructured highly porous composite collagen sponge, the nanofiber collagen layer is prepared first as in Example 20. Stabilization, freezing and lyophilization are also performed as in Example 20. Subsequently, the six layers thus prepared are stacked and placed in 0.8% w/w collagen dispersion for 3 hours at 20 °C. Under these conditions, the nanofiber layers are saturated with the collagen dispersion, followed by freezing at -80 °C for 4 hours and lyophilization until a constant weight is reached. After lyophilization, the stability of the collagen composite sponge is increased by dipping in a solution of 95% w/w ethanol and water with EDC and NHS at 30 °C for 3 hours. 1.40 g of EDC and 0.350 g of NHS mixed with 150 ml of 95% w/w ethanol solution with water were used per 1 g of collagen. After collagen crosslinking, the sponge is washed with 1.2 M disodium hydrogen phosphate for at least 30 minutes and further washed for at least 20 minutes in distilled water, frozen at -30 °C for 6 hours and lyophilized. The volume ratio of all fiber layers to dispersion is 1: 4, and therefore the volume ratio of fiber layers and sponge in the resulting composite is 1: 4, i.e., the final volume amount of collagen nanofiber layers in the composite is 20%. The last step of the preparation is the impregnation of the composite sponge with 95% w/w a solution of ethanol with water containing a weighed amount of gentamicin so that its final amount is 50% w/w. The impregnated composite is then frozen at -25 °C for 6 hours and lyophilized. In this way, a composite sponge is obtained with a final amount of collagen of 50% w/w and 50% w/w of antibiotics.

### Industrial applicability

Degradable collagen sponges with hemostatic effects, controlled degradation time and controlled local release of antibiotics according to this invention can be used in human and veterinary medicine, especially in surgery, orthopedics, traumatology and plastic surgery. The sponge itself is suitable for use in cases where the terrain is potentially infectious, for example where there is a risk of infection during surgery or treatment of an open wound or ulcer. In some cases, the collagen dressing alone can be used due to its hemocoagulating effects. The addition of active substances, such as antibiotics, then serves as a quick, short-term prevention. The degradation time is also faster. Sandwich collagen sponges with a low porous core and highly porous peripheral layers with hemostatic effects, controlled degradation time and controlled local release of antibiotics according to this invention can be used in human and veterinary medicine, especially in surgery, orthopedics, wound dressings, treatment of leg ulcers, traumatology and plastic surgery. It is particularly suitable for less extensive inflammation or in potentially infectious terrain, i.e., where first a rapid release of the optimal dose of active substance is required, and then a longer-lasting effect of the active substance, which prevents the recurrence of inflammation.

## Claims

1. A method of preparation of the sandwich collagen sponge comprising peripheral layers formed by a highly porous sponge which contains type I collagen and a low porous core containing type I collagen, **characterized in that** 4 to 8% w/w dispersion of type I collagen in water is first allowed to swell at 5-15 °C for 1-3 hours, then the mixture is homogenized, after homogenization the mixture is dispensed into moulds, frozen at -60 to -90 °C for 3 - 8 hours and lyophilized to form a low porous core, which is subsequently impregnated with 0.1 - 3% w/w dispersion of type I collagen for 1-3 hours at 15-25 °C, then the mixture is frozen again at -60 to -90 °C for 3 - 8 hours and lyophilized to form a sandwich collagen sponge, after which the sponge is stabilized by chemical crosslinking which is carried out by dipping the sponge in a solution of 95% w/w ethanol and water with N-(3-dimethylaminopropyl)-N-ethylcarbodiimide hydrochloride and N-hydroxysuccinimide at 25 - 40 °C for 2 - 4 hours, washed, frozen at -15 to -30 °C for 5 - 7 hours and lyophilized.

2. The method of preparation of the sandwich collagen sponge according to claim 1, **characterized in that** in crosslinking 0.5-1.5 g of N-(3-dimethylaminopropyl)-N-ethylcarbodiimide hydrochloride and 0.125 -0.750 g of N-hydroxysuccinimide is used per 1 g of collagen.

3. The method of preparation of the sponge according to claim 1, **characterized in that** after the last lyophilization the sponge is impregnated with 95% w/w solution of ethanol with water containing one or more active substances, preferably selected from the group including an antibiotic, an anesthetic, an antiaggregant, an antiviral, or a mixture thereof, preferably an antibiotic.

4. The method of preparation of the sponge according to claim 3, **characterized in that** the ratio of the collagen to the total amount of active substances in the collagen sponge is 40 - 70% w/w of collagen to 30 - 60% w/w of active substances.

5. A sandwich collagen sponge for controlled release of active substances produced by the method according to any of the preceding claims.

## Patentansprüche

1. Verfahren zur Vorbereitung eines verbundplattenartigen Kollagenschaums, der aus einem hochporösen, ein Kollagen vom Typ I enthaltenden Schaum bestehende Randschichten sowie einen niederporösen, ein Kollagen vom Typ I enthaltenden Kern umfasst, **dadurch gekennzeichnet, dass** zunächst eine Dispersion des Kollagens vom Typ I in der Menge von 4 bis 8 Gew.-% im Wasser bei einer im Bereich von 5 bis 15 °C liegenden Temperatur 1 bis 3 Stunden lang quellen gelassen wird, im Anschluss daran das derart gewonnene Gemisch homogenisiert wird und nach der Homogenisierung das Gemisch in geeignete Formen eindosiert wird, in den letzteren bei einer im Bereich von -60 bis -90 °C liegenden Temperatur 3 bis 8 Stunden lang eingefroren wird und daraufhin unter Bildung des niederporösen Kerns lyophilisiert wird, welcher Kern anschliessend bei einer im Bereich von 15 bis 25 °C liegenden Temperatur 1 bis 3 Stunden lang durch die Einwirkung einer Dispersion des Kollagens vom Typ I in der Menge von 0,1 bis 3 Gew.-% imprägniert wird, woraufhin das derart aufbereitet Gemisch wieder bei einer im Bereich von -60 bis -90 °C liegenden Temperatur 3 bis 8 Stunden lang eingefroren und unter Bildung eines verbundplattenartigen Kollagenschaums lyophilisiert wird, welcher Schaum anschliessend durch chemische Vernetzung stabilisiert wird, die durch das Einweichen des Schaums in einer 95 Gew.-% Ethanol und Wasser enthaltenden Lösung unter Zugabe von N-(3-Dimethylaminopropyl)-N-ethylcarbodiimid-Hydrochlorid und N-Hydroxysuccinimid bei einer im Bereich von 25 bis 40 °C liegenden Temperatur 2 bis 4 Stunden lang erfolgt, und anschliessend durchgewaschen und daraufhin bei einer im Bereich von -15 bis -30 °C liegenden Temperatur 5 bis 7 Stunden lang eingefroren und lyophilisiert wird.

2. Verfahren zur Vorbereitung eines verbundplattenartigen Kollagenschaums nach Anspruch 1, **dadurch gekennzeichnet, dass** während der Vernetzung N-(3-Dimethylaminopropyl)-N-ethylcarbodiimid-Hydrochlorid in der Menge von 0,5-1,5 g und N-Hydroxysuccinimid in der Menge von 0,125 - 0,750 g per 1 g Kollagen zugegeben wird.

3. Verfahren zur Vorbereitung eines Schaums nach Anspruch 1, **dadurch gekennzeichnet, dass** der Schaum nach der als letzte erfolgten Lyophilisierung und Einwirkung einer 95 Gew.-% Ethanol und Wasser enthaltenden Lösung unter Zugabe von einer oder mehreren aktiven Substanzen imprägniert wird, welche Substanzen vorteilhafterweise aus der Gruppe ausgewählt sind, die ein Antibiotikum, Anästhetikum, Antiaggregans, Antivirotikum oder deren Gemische, insbesondere ein Antibiotikum, umfasst.

4. Verfahren zur Vorbereitung eines Schaums nach Anspruch 3, **dadurch gekennzeichnet, dass** das Verhältnis zwischen der Menge an Kollagen und der Gesamtmenge an die in dem Kollagenschaum enthaltenen aktiven Substanzen bei 40 bis 70 Gew.-% von Kollagen gegenüber 30 bis 60 Gew.-% von aktiven Substanzen liegt.

5. Ein verbundplattenartiges Kollagenschaum zur kontrollierten Freisetzung von aktiven Substanzen, hergestellt in einem Verfahren nach einem der vorhergehenden Ansprüche.

## Revendications

1. Procédé de préparation d'une éponge sandwich de collagène comprenant des couches périphériques formées par une éponge hautement poreuse qui comprend du collagène de type 1 et un noyau faiblement poreux comprenant du collagène de type I,
**caractérisé en ce qu'**une dispersion de 4 à 8% p/p de collagène de type I dans l'eau est laissée à gonfler à une température de 5 à 15 °C pendant 1 à 3 heures, puis le mélange est homogénéisé, après la homogénéisation le mélange est distribué dans des moules, gelé à une température de -60 à -90 °C pendant 3 à 8 heures et lyophilisé afin de former un noyau faiblement poreux qui est ensuite imprégné d'une dispersion de 0.1 à 3% p/p de collagène de type 1 pendant 1 à 3 heures à une température de 15 à 25 °C, puis le mélange est gelé à nouveau à une température de -60 à -90 °C pendant 3 à 8 heures et lyophilisé afin de former une éponge sandwich de collagène, après quoi l'éponge est stabilisée par la réticulation chimique qui est effectuée par l'immersion de l'éponge dans une solution de 95% p/p d'éthanol et d'eau avec du N-(3-diméthylaminopropyl)-N-éthylcarbodiimide chlorhydrate et du N-hydroxysuccinimide à une température de 25 à 40 °C pendant 2 à 4 heures, lavée, gelée à une température de -15 à -30 °C pendant 5 à 7 heures et lyophilisée.

2. Procédé de préparation d'une éponge sandwich de collagène selon la revendication 1, **caractérisé en ce que** dans la réticulation 0.5 à 1.5 g de N-(3-diméthylaminopropyl)-N-éthylcarbodiimide chlorhydrate et 0.125 à 0.750 g de N-hydroxysuccinimide est utilisé pour 1 g de collagène.

3. Procédé de préparation d'une éponge selon la revendication 1, **caractérisé en ce qu'**après la dernière lyophilisation l'éponge est imprégnée de solution de 95% p/p d'éthanol avec de l'eau comprenant une ou plusieurs substances actives, préférablement sélectionnées dans le groupe qui comprend un antibiotique, un anesthésique, un antiagrégant, un médicament antiviral, ou un mélange de ceux-ci, préférablement un antibiotique.

4. Procédé de préparation d'une éponge selon la revendication 3, **caractérisé en ce que** le rapport entre le collagène et la quantité totale des substances actives dans l'éponge de collagène est 40 à 70% p/p de collagène à 30 à 60% p/p de substances actives.

5. Éponge sandwich de collagène pour une libération contrôlée des substances actives produite par le procédé selon l'une quelconque des revendications précédentes.
